# EUROPEAN PATENT SPECIFICATION

(11) **EP 1 310 241 B1**
(45) Date of publication and mention of the grant of the patent: **01.03.2006**
(21) Application number: 02024802.7
(22) Date of filing: 07.11.2002
(51) Int. Cl.: A61K 8/58

(54) **Skin treatment using a retinoid**
Hautbehandlung beim Gebrauch eines Retinoids
Traitement cutané utilisant un rétinoide

(30) Priority: 09.11.2001 US 39745; 05.11.2002 US 288603
(43) Date of publication of application: 14.05.2003
(73) Proprietor: AVON PRODUCTS, INC., New York, NY 10020-1196 (US)
(72) Inventor: Duggan, Michele, Middletown, New York 10940 (US); Cowton, Lorraine M., Cornwall, New York 12518 (US); Duffy, John, West Milford, New Jersey 07480 (US)
(74) Representative: Dr. Weitzel & Partner

(56) References cited:
- US-A- 4 804 670
- US-A- 5 760 276
- US-A- 6 166 244

## Description

### BACKGROUND OF THE INVENTION

### 1. Field of the Invention

The present invention relates to the use of a novel retinoid, particularly a retinol derivative. More particularly, the present invention relates to the use of topical compositions having the retinol derivative, retinoxytrimethylsilane, to treat, including prevent, ameliorate and/or reduce, signs of dermatological aging, especially wrinkles, and/or improve the aesthetic appearance of skin. Still more particularly, the present invention provides the beneficial effects of retinol without causing irritation from daily use. The present invention may also be used to improve the aesthetic appearance of lips (e.g. by decreasing the number and/or depth of vertical lip lines).

### 2. Description of the Prior Art

Retinoid or retinoid compounds/derivatives (collectively referred to herein as "retinoid" or "retinoids") are vitamin A derivatives. They are used in topical compositions to treat a variety of adverse skin conditions. Such skin conditions include acne, actinic damage, dandruff, eczema, fine lines, psoriasis, warts and wrinkles.

Retinoids used in the prior art include, but are not limited to, isotretinoin, retinal, retinol, retinoic acid, retinyl acetate, retinyl palmitate, retinyl propionate, synthetic retinoid mimics, and tretinoin. As is known in the art, the amount of retinoid in a topical composition varies depending on the condition to be treated, as well as on the composition and the retinoids themselves. Representative compositions having a retinoid are discussed, by way of example, in U.S. Patent Nos. 3,006,939; 3,060,229; 3,932,665; 4,826,828; and 4,934,114.

However, topical compositions having a retinoid have been limited in the amount of retinoid, since retinoid has been found to irritate the skin. Such irritation is acute, especially when the amount of retinoid in the composition is high. However, some consumers with sensitive skin cannot even tolerate a small amount of retinoid.

The irritation can manifest itself in the form of physical discomfort and/or unaesthetic skin appearance. Such an unaesthetic skin appearance can manifest itself by dermatitis or erythema. The irritation may disturb the user to such an extent that the user will discontinue use of the composition having a retinoid. Alternately, the user may reduce the frequency of use, thereby possibly reducing the effectiveness of the composition for its intended purpose.

Heretofore, there has not been an efficacious skin care composition that contains a retinoid, but without the irritation induced by a retinoid. Historically, it has been observed that retinoid-induced skin irritation has a direct correlation to retinoid efficacy. Namely, the greater the concentration of retinol, the more likely that irritation will occur. Thus, simply reducing the retinoid concentration might decrease irritation to the skin, but it would also reduce the desired retinoid efficacy. Consumers require, and would benefit from, a retinol-delivering composition that is both well-tolerated and efficacious. The present composition having the retinoid, retinoxytrimethylsilane, achieves this need.

### SUMMARY OF THE INVENTION

It is an object of the present invention to provide a topical composition having retinoxytrimethylsilane to treat, including prevent, ameliorate and/or reduce, signs of dermatological aging, especially wrinkles, and/or improve the aesthetic appearance of skin, and methods of its use.

It is another object to provide such a topical composition that is substantially anhydrous.

It is a further object of the present invention to provide a method for delivering the benefits of retinol without inducing irritation.

It is yet a further object of the present invention to provide a composition and method that can be used to re-moisturize the lip, improve lip color, lip clarity, lip dryness and/or reduce the number and depth of lip lines.

These and other objects and advantages of the present invention are achieved by a topical composition that has retinoxytrimethylsilane in an effective amount. When the topical composition is intended for application to the skin, the retinoxytrimethylsilane is preferably present from about 0.1 weight percent (wt%) to about 50 wt% of the total weight of the composition.

### DETAILED DESCRIPTION OF THE INVENTION

The present invention relates to the surprising recognition that retinoxytrimethylsilane, a retinol derivative, delivers retinoid efficacy without skin irritation, dryness or erythema typically caused by the topical use of a retinoid. Further, it has also been discovered in the present invention that moisture-activated, retinol-delivering compositions having retinoxytrimethylsilane will treat, including prevent, ameliorate, and/or reduce, signs of dermatological aging, especially wrinkling and/or improve the aesthetic appearance of the skin and/or lips upon daily application. The skin composition is preferably applied topically once or twice daily. The daily application to skin can be for periods up to two weeks, four weeks or more, but preferably is for at least two weeks.

The present skin compositions preferably have an amount of retinoxytrimethylsilane from about 0.1 wt% to about 50 wt%, based on the total weight of the composition. More preferably, the present skin compositions have retinoxytrimethylsilane in an amount from about 0.5 wt% to about 15 wt%. Most preferably, the present skin compositions have retinoxytrimethylsilane in an amount from about 0.5 wt% to about 5 wt% of the total composition. To provide guidance to those skilled in the art for practicing the present invention, the efficacy of the retinol-derivative concentration in a topical composition is approximately equivalent to 10⁻¹ concentration of retinol percent by weight of the composition. For example, to provide the efficacy of about 0.1 wt% (based upon the total weight of the composition) retinol activity, a composition of the present invention preferably includes about 1 wt% of retinoxytrimethylsilane.

When the composition is a lip composition, the concentration of retinoxytrimethylsilane may be modified by those in the art to reflect the intended use of the lip composition. For example, when the lip composition is expected or intended to be applied multiple times a day, e.g. when the lip composition is a colored/pigmented lip stick/gloss or a lip balm, the concentration of retinoxytrimethylsilane may be less then when the lip product is intended for application only once daily. For example, a suitable pigmented lipstick according to the present invention preferably has greater than about 0.01 wt%, more preferably greater than 0.015 wt%, and most preferably greater than about 0.2 wt% retinoxytrimethylsilane. In addition, when the lip product is intended to be applied multiple times a day, it is preferred that the lip product includes less than about 0.75 wt%, and more preferably less than about 0.5 wt%, retinoxytrimethylsilane.

In addition to improvements in skin and lip care benefits of the present invention, the retinol-derivative, retinoxytrimethylsilane, results in more stable compositions than those of the prior art. This is evidenced by a lasting white color of the present compositions, without the manifestation of yellowing over time, particularly in a stick product form. The present compositions can also be in other cosmetic product forms, such as, for example, an emulsion, a gel, a lip balm, a lip gloss, a lipstick, a lotion, a mask, an ointment, a patch, a pomade, a solution, a spray, or a towelette.

The present compositions are preferably wax-based, oil-based, or silicone-based compositions, all of which are substantially anhydrous (i.e., contain less than about 1 wt%, more preferably no, water). Alternatively, the compositions may include greater than 1 wt% water as long as contact between the retinoxytrimethylsilane and water is prevented. For example, a two-phase product that is dispensed in a dual-chamber package with each phase in a separate chamber may include an aqueous phase in one chamber and the retinoxytrimethylsilane in substantially anhydrous phase in the other chamber. As the contents from each chamber are dispensed, the phases may combine either via the dispensing mechanism, as is known in the art, or mixed by the user as the user rubs the two phases onto and into the skin. Correspondingly, when the present invention is a lip composition, it is preferred that the composition be anhydrous. However, a dual-chamber package or a non-anhydrous overcoat may be used to couple the benefits of the present invention with aqueous compositions or components.

In a preferred embodiment of the present invention, the retinoxytrimethylsilane used is in soybean oil and is commercially available from Clariant Corp. under the tradename SilCare 1 M 75. The Clariant product is a 12% chemically reactive liquid derivative of retinol, which is readily dispersible in most substantially anhydrous organic cosmetic formulations at room temperature.

The compositions of the present invention yield improvements to the aesthetic appearance of the skin by treating, including preventing, ameliorating and/or reducing at least one of the following: dermatological aging, especially chronological, actinic or hormonal aging, skin fragility, loss of collagen and/or elastin, skin atrophy, skin dryness, skin flakiness, skin discoloration, skin sagging, signs of skin fatigue and/or stress. In particular, improvements to the aesthetic appearance of skin include at least one of the following: makes facial lines appear less noticeable, makes facial lines and/or wrinkles feel plumped, improves appearance of suborbital lines and/or periorbital lines, improves appearance of crow's feet, reduces and/or diminishes the appearance of wrinkles, particularly facial wrinkles on the cheeks, forehead and/or around the mouth, and particularly deep wrinkles, rejuvenates and/or revitalizes skin, particularly aging skin, improves skin firmness and/or plumpness, improves skin tone, enhances skin thickness, decreases pore size, improves skin moisture and/or hydration, improves skin texture, reduces and/or eliminates fine and/or deep lines, smoothes skin, restores skin luster and/or brightness, and improves skin resiliency, flexibility and/or elasticity.

When the compositions of the present invention are in the form of a product that can be applied to the lip, the improvement in aesthetic appearance of the lip includes at least one of the following: reduces, ameliorates and/or treats the signs of chronological/photo aging by improving the appearance of wrinkled lips; decreases epithelial fragility of the lip; ameliorates, forestalls and/or reverses loss of collagen; prevents epithelial atrophy of the lip; promotes/accelerates cell turnover; improves lip/epithelial firmness/plumpness; improves lip/epithelial texture; decreases / minimizes the appearance of fine lines (particularly vertical); decreases size and/or depth of wrinkles (particularly vertical); improves lip/epithelial tone; enhances lip/epithelial thickness; increases moisture retention; minimizes lip/epithelial discoloration; or reverses age-associated cornification of lip epithelia.

A preferred embodiment of the topical compositions of the present invention also includes at least one of the following: a surface smoother, a skin plumper, an optical diffuser, a sunscreen, an exfoliation promoter, and an antioxidant.

A preferred embodiment of the lip composition of the present invention also includes at least one of following: a pigment (e.g., iron oxides, a wax (e.g., carnauba, candela, ceresine wax and/or petroleum wax), silica, titanium dioxide, oils (e.g., silicone or soybean), sunscreen, an antioxidant (e.g., tocopherol), or a film former.

A surface smoother provides the functional benefits of enhancing skin smoothness and reducing the appearance of fine lines and coarse wrinkles. Examples include silicas, talcs, isopropyl myristate, petrolatum, isopropyl lanolate, silicones (e.g., methicone, dimethicone), polymethylmethacrylate (PMMA), or any mixtures thereof. The surface smoother is preferably present from about 0.1 wt% to about 50 wt% of the total weight of the composition.

A skin plumper serves as a collagen enhancer to the skin. An example of a suitable, and preferred, skin plumper is palmitoyl oligopeptide. Other skin plumpers are collagen and/or glycosaminoglycan (GAG) enhancing agents. The skin plumper is preferably present from about 0.1 wt% to about 20 wt% of the total weight of the composition.

An optical diffuser is a particle that changes the surface optometrics of skin, resulting in a visual blurring and softening of, for example, lines and wrinkles. Examples of optical diffusers that can be used in the present invention include, but are not limited to, boron nitride, mica, nylon, polyurethane powder, sericite, silica, silicone powder, talc, Teflon, titanium dioxide, zinc oxide, or any mixtures thereof. The optical diffuser is preferably present from about 0.01 wt% to about 20 wt% of the total weight of the composition.

A sunscreen protects the skin from damaging ultraviolet rays. In an illustrative embodiment of the present invention, the sunscreen would provide both UVA and UVB protection, by using either a single sunscreen or a combination of sunscreens. Among the sunscreens that can be employed in the present compositions are avobenzone, cinnamic acid derivatives (such as octylmethoxy cinnamate), octyl salicylate, oxybenzone, titanium dioxide, zinc oxide, or any mixtures thereof. Preferably, the sunscreen is present from about 1 wt% to about 30 wt% of the total weight of the composition. In particular, the addition of a sunscreen is preferred to prevent/reduce the photodegradation of retinoid while in the package and/or on the skin.

The compositions of the present invention having sunscreen bring about additional improvements to the aesthetic appearance of skin, including at least one of the following: minimizes sunburning, minimizes tanning, and reduces redness.

The present compositions may also have one or more exfoliation promoters. Suitable examples of an exfoliation promoter that can be used in the present compositions include alpha hydroxy acids; benzoyl peroxide; beta hydroxy acids; keto acids, such as pyruvic acid, 2-oxopropanoic acid, 2-oxobutanoic acid, and 2-oxopentanoic acid; oxa acids as disclosed in U.S. Patent Nos. 5,847,003 and 5,834,513 (the disclosures of which are incorporated herein by reference); salicylic acid; stearoxytrimethylsilane (available from Clariant under the tradename SILCARE 1 M 71); trimethylsilyl trimethylsiloxy glycolate (available from Clariant under the trade name SILCARE 180 M 30); trimethylsilyl trimethylsiloxy lactate (available from Clariant under the tradename SILCARE 180 M 10); urea; or any mixtures thereof. The preferred exfoliation promoters are 3,6,9-trioxaundecanedioic acid, glycolic acid, lactic acid, or any mixtures thereof.

When the present invention includes an exfoliation promoter, the composition has about 1 wt% to 20 wt%, preferably about 1 wt% to about 15 wt%, more preferably about 4 wt% to about 10 wt%, and most preferably about 4 wt%, of the exfoliation promoter based on the total weight of the composition.

An antioxidant functions, among other things, to scavenge free radicals from skin to protect the skin from environmental agressors. Examples of antioxidants that may be used in the present compositions include compounds having phenolic hydroxy functions, such as ascorbic acid and its derivatives/esters; beta-carotene; catechins; curcumin; ferulic acid derivatives (e.g. ethyl ferulate, sodium ferulate); gallic acid derivatives (e.g. propyl gallate); lycopene; reductic acid; rosmarinic acid; tannic acid; tetrahydrocurcumin; tocopherol and its derivatives; uric acid; or any mixtures thereof. Other suitable antioxidants are those that have one or more thiol functions (-SH), in either reduced or non-reduced form, such as glutathione, lipoic acid, thioglycolic acid, and other sulfhydryl compounds. The antioxidant may be inorganic, such as bisulfites, metabisulfites, sulfites, or other inorganic salts and acids containing sulfur. Compositions of the present invention may have an antioxidant preferably from about 0.001 wt% to about 10 wt%, and more preferably from about 0.001 wt% to about 5 wt%, of the total weight of the composition.

The present composition may also have one or more of the following ingredients or adjuvants: anesthetics, anti-allergenics, antifungals, antiseptics, chelating agents, colorants, demulcents, emollients, emulsifiers, fragrances, humectants, lubricants, moisturizers, pH adjusters, pigment altering agents, preservatives, skin penetration enhancers, stabilizers, surfactants, thickeners, viscosity modifiers, vitamins, or any mixtures thereof.

The component(s) of the present invention are preferably contained in a cosmetically acceptable vehicle. Suitable vehicles of the present invention may also include mineral oil, petrolatum, polydecene, and vegetable oil.

To form the exemplary wax-based stick products of the present invention, processing is undertaken at typical temperatures for that type of base (e.g., about 150 °F to about 170 °F), over extended periods of time. Higher temperatures are feasible due to greater stability of the retinol-derivative of the current invention as compared to active retinol. Also, processing at room temperature is a suitable means for forming the compositions of the present invention. In short, the present compositions can be processed from room temperature just up to about 180 °F, even though about 150 °F to about 170 °F is preferred.

The following is an illustrative, non-limiting example of a composition of the present invention.

### Example

A facial treatment stick composition of the present invention was prepared comprising the following ingredients: about 0.1 wt% retinoxytrimethylsilane, soja (soybean) oil, methicone, PPG-5-ceteth-20, octyl methoxycinnamate, palmitoyl oligopeptide, zinc oxide, titanium dioxide, petrolatum, methylparaben, aluminum hydroxide, polydecene, benzoic acid, isopropyl myristate, isopropyl lanolate, ozokerite, tocopheryl acetate, and silica.

This inventive composition was compared to two products: (1) a wrinkle cream available from Johnson & Johnson under the combined tradename ROC™ ACTIF PUR™ and (2) a generic retinol cream. The ROC™ product has the following ingredients: about 0.1 wt% retinol, water, octyl hydroxystearate, ceteareth-20, glycerin, glyceryl distearate, dimethicone, C12-15 alkyl lactate, steareth-10, stearyl alcohol, sodium citrate, cholesterol, cetearyl alcohol, acetylated lanolin, phenoxyethanol, polysorbate 80, methylparaben, polyacrylamide, disodium EDTA, propylparaben, cetyl acetate, laureth-7, polysorbate 20, BHT, C13-14 isoparaffin, ascorbic acid, and BHA. The generic retinol cream has the following ingredients: about 0.1 wt% retinol, water, carbopol 934, disodium EDTA, veegum, glycerin, propylene glycol, emulsifying wax, cetyl ricinoleate, cetyl alcohol, C10-30 cholesterol/lanosterol esters, stearic acid, POE (10M) soya sterol, methylparaben, triethanolamine, dimethyl polysiloxane, and benzyl alcohol.

There was a two-week, double-blind, split-face, baseline controlled study conducted at an independent laboratory on a population with clinically sensitive skin, in which each composition was applied once daily. This study compared the composition of the present invention to the two products identified above.

Forty women participated in this study. The forty women ranged in age from 29 to 64 years. The panelists were required to have clinically sensitive skin (i.e., atopic and/or rosacea) background. Dermatological examinations for irritation attributes were conducted at baseline, one and two weeks. Each panelist applied all three products on their face once daily in the morning. The test products were identified to enhance compliance.

Table 1 indicates that the composition of the present invention significantly improved dryness and erythema (redness) at two weeks, while ROC™ and the generic retinol cream significantly worsened both conditions (indicated by a negative number). In addition, the composition of the present invention improved the condition of papules/pustules as compared to the generic retinol cream, and significantly improved the same condition as compared to the ROC™ product, which instead significantly worsened the condition.

A further aspect of the present invention is the use of retinoxytrimethylsilane for the manufacture of a substantially anhydrous composition having retinoxytrimethylsilane wherein the composition is applied to the skin and wherein the improvement in aesthetic appearance of skin is the treatment of at least one condition selected from acne, psoriasis and warts.

**Table 1**

| Comparison of Magnitude of Change from baseline in Dermatologist Score | | | |
|---|---|---|---|
| Improvement Parameter | Present Invention % Δ @ Week 2 | Roc % Δ @ Week 2 | Generic Cream % Δ @ Week 2 |
| Dryness | 26% | -38% | -15% |
| Erythema | 15% | -19% | -17% |
| Papules/Pustules | 50% | -33% | 33% |

As Table 2 shows, the dermatologist observed that at two weeks, dryness either decreased or remained the same in all panelists using the composition of the present invention, while 73% of the subjects using ROC™ showed an increase in dryness. Thirty-three percent of the panelists using the generic retinol cream also had an increase in dryness. Ninety-three percent of the panelists using the composition of the present invention showed either a decrease or no change in erythema (redness), while 42% of panelists using ROC™ and 37% of panelists using the generic retinol cream showed increased erythema.

**Table 2**

| % of Panelist Improving or Declining | | | |
|---|---|---|---|
| Improvement Parameter | Present Invention % Δ @ Week 2 | Roc % Δ @ Week 2 | Generic Cream % Δ @ Week 2 |
| Decrease in Dryness | 54% | 8% | 15% |
| Increase in Dryness | 0 | 73% | 33% |
| Dryness Remained the Same | 46% | 19% | 52% |
| | | | |
| Decrease in Erythema | 41% | 8% | 0% |
| Increase in Erythema | 7% | 42% | 37% |
| Erythma Remained the Same | 52% | 50% | 63% |

As is evident, the composition of the present invention significantly improved erythema and dryness after two weeks of use, whereas ROC™ and the generic retinol cream significantly exacerbated both conditions. This is indicated by both magnitude of change and percent of panel improving or declining. These are impressive findings because increases in skin surface texture changes (dryness) and erythema (redness) are typical in the first 2 to 3 weeks of topical retinoid use. In addition, all of the panelists were clinically sensitive-skinned individuals.

Thus, the composition of the present invention decreased dryness and erythema (redness), while the ROC™ and generic retinol cream significantly increased the same attributes. Therefore, the composition of the present invention is superior to the prior art for treating sensitive skin.

It should be understood that the foregoing description is only illustrative of the present invention. Various alternatives and modifications can be devised by those skilled in the art without departing from the invention. Accordingly, the present invention is intended to embrace all such alternatives, modifications and variations that fall within the scope of the appended claims.

## Claims

1. A method of treating fine lines and/or wrinkles, comprising applying to skin a topical, substantially anhydrous composition having an amount of retinoxytrimethylsilane effective to prevent, ameliorate and/or reduce fine lines and/or wrinkling.

2. The method of claim 1, wherein said composition is applied for a period of time effective to prevent, ameliorate and/or reduce fine lines and/or wrinkling.

3. The method of claim 2, wherein said applying of the composition occurs once or twice daily up to two or four weeks.

4. The method of claim 1, wherein said retinoxytrimethylsilane is present in an amount from about 0.1 wt% to about 50 wt% of the total weight of the composition.

5. The method of claim 1, wherein said retinoxytrimethylsilane is present in an amount from about 0.5 wt% to about 15 wt% of the total weight of the composition.

6. The method of claim 1, wherein said retinoxytrimethylsilane is present in an amount from about 0.5 wt% to about 5 wt% of the total weight of the composition.

7. The method of claim 1, wherein said composition further comprises a cosmetically acceptable vehicle.

8. A method of improving the aesthetic appearance of skin or lip comprising topically applying to the skin or lip a substantially anhydrous composition having retinoxytrimethylsilane in an amount effective to improve the aesthetic appearance of the skin or lip.

9. The method of claim 8, wherein the composition is applied to the skin and wherein the improvement in aesthetic appearance is selected from the group consisting of makes facial lines appear less noticeable, makes facial lines and/or wrinkles feel plumped, improves appearance of suborbital lines and/or periorbital lines, improves appearance of crow's feet, reduces and/or diminishes the appearance of wrinkles, rejuvenates and/or revitalizes skin, improves skin firmness and/or plumpness, improves skin tone, enhances skin thickness, decreases pore size, improves skin moisture and/or hydration, improves skin texture, reduces and/or eliminates fine and/or deep lines, smoothes skin, restores skin luster and/or brightness, and improves skin resiliency, flexibility and/or elasticity.

10. The method of claim 8, wherein the composition is applied to the skin and wherein the improvement in aesthetic appearance of skin is the treatment of at least one condition selected from the group consisting of dermatological aging, skin fragility, loss of collagen and/or elastin, skin atrophy, skin dryness, skin flakiness, skin sagging, skin discoloration, signs of skin fatigue and/or stress.

11. The method of claim 10, wherein said dermatological aging is selected from the group consisting of chronological aging, actinic aging, hormonal aging, or any combinations thereof.

12. The method of claim 8, wherein said skin is sensitive skin.

13. The method of claim 8, wherein said appearance of skin is substantially free of skin irritation.

14. The method of Claim 8, wherein the composition is applied to the lip and wherein improvement in aesthetic appearance of the lip includes at least one of the group consisting of reduces, ameliorates and/or treats the signs of chronological/photo aging by improving the appearance of wrinkled lips; decreases epithelial fragility of the lip; ameliorates, forestalls and/or reverses loss of collagen; prevents epithelial atrophy of the lip; promotes/accelerates cell turnover; improves lip/epithelial firmness/plumpness; improves lip/epithelial texture; decreases / minimizes the appearance of fine lip lines, particularly fine vertical lip lines; decreases size and/or depth of wrinkles, particularly vertical lip wrinkles; improves lip/epithelial tone; enhances lip/epithelial thickness; increases moisture retention; minimizes lip/epithelial discoloration; and reverses age-associated cornification of lip epithelia.

15. The method of Claim 8 wherein the composition is applied to the lip and wherein improvement in aesthetic appearance of the lip includes at least one of the group consisting of reduces, ameliorates and/or treats the signs of chronological/photo aging by improving the appearance of wrinkled lips; improves lip/epithelial firmness/plumpness; improves lip/epithelial texture; decreases / minimizes the appearance of vertical fine lip lines; decreases size and/or depth of vertical lip wrinkles; improves lip/epithelial tone; enhances lip/epithelial thickness; increases moisture retention; and minimizes lip/epithelial discoloration.

16. A method of improving the aesthetic appearance of skin comprising: topically applying to skin, a substantially anhydrous composition having retinoxytrimethylsilane, in an amount and for a period of time sufficient to improve the aesthetic appearance of skin.

17. The method of claim 16, wherein said applying of the composition occurs once daily or twice daily up to two or four weeks.

18. A topical, substantially anhydrous composition, comprising: retinoxytrimethylsilane in an amount effective to prevent, ameliorate, reduce and/or treat fine lines and/or wrinkles.

19. The composition of claim 18, wherein said retinoxytrimethylsilane is present in an amount from about 0.1 wt% to about 50 wt% of the total weight of the topical composition.

20. The composition of claim 18, wherein said retinoxytrimethylsilane is present in an amount from about 0.5 wt% to about 15 wt% of the total weight of the composition.

21. The composition of claim 18, wherein said retinoxytrimethylsilane is present in an amount from about 0.5 wt% to about 5 wt% of the total weight of the composition.

22. The composition of claim 18, further comprising a cosmetically acceptable vehicle.

23. The composition of claim 18, further comprising an antioxidant.

24. The composition of claim 18, further comprising a sunscreen.

25. The composition of claim 18, wherein the composition is a lip composition and said retinoxytrimethylsilane is present in an amount effective to prevent, ameliorate, reduce and/or treat fine lines and/or wrinkles.

26. The composition of claim 25, wherein said lip composition has at least about 0.01 wt% of said retinoxytrimethylsilane.

27. The composition of claim 25, wherein said lip composition has at least about 0.015 wt% of said retinoxytrimethylsilane.

28. The composition of claim 25, wherein said lip composition has at least about 0.02 wt% of said retinoxytrimethylsilane.

29. The composition of claim 25, wherein said lip composition has less than about 0.75 wt% of said retinoxytrimethylsilane.

30. The composition of claim 25, wherein said lip composition has less than about 0.50 wt% of said retinoxytrimethylsilane.

31. Use of retinoxytrimethylsilane for the manufacture of a substantially anhydrous composition having retinoxytrimethylsilane wherein the composition is applied to the skin and wherein the improvement in aesthetic appearance of skin is the treatment of at least one condition selected from acne, psoriasis, eczema and warts.

## Patentansprüche

1. Verfahren zur Behandlung feiner Falten und/oder Runzeln, umfassend das Auftragen einer topischen, im Wesentlichen wasserfreien Zusammensetzung auf die Haut, wobei diese Zusammensetzung eine Menge an Retinoxytrimethylsilan enthält, die wirksam feine Falten und/oder Runzeln verhindert, verbessert und/oder reduziert.

2. Verfahren nach Anspruch 1, wobei die Zusammensetzung über einen Zeitraum aufgetragen wird, so dass feine Falten und/oder Runzeln wirksam verhindert, verbessert und/oder reduziert werden.

3. Verfahren nach Anspruch 2, wobei das Auftragen der Zusammensetzung ein- oder zweimal täglich für bis zu zwei oder vier Wochen erfolgt.

4. Verfahren nach Anspruch 1, wobei Retinoxytrimethylsilan in einer Menge von etwa 0,1 Gew.% bis etwa 50 Gew.% des Gesamtgewichts der Zusammensetzung vorhanden ist.

5. Verfahren nach Anspruch 1, wobei Retinoxytrimethylsilan in einer Menge von etwa 0,5 Gew.% bis etwa 15 Gew.% des Gesamtgewichts der Zusammensetzung vorhanden ist.

6. Verfahren nach Anspruch 1, wobei Retinoxytrimethylsilan in einer Menge von etwa 0,5 Gew.% bis etwa 5 Gew.% des Gesamtgewichts der Zusammensetzung vorhanden ist.

7. Verfahren nach Anspruch 1, wobei die Zusammensetzung darüber hinaus einen kosmetisch akzeptablen Träger enthält.

8. Verfahren zur Verbesserung des ästhetischen Erscheinungsbilds der Haut oder der Lippen, umfassend das topische Auftragen einer im Wesentlichen wasserfreien Zusammensetzung auf die Haut oder die Lippen, wobei diese Zusammensetzung Retinoxytrimethylsilan in einer Menge enthält, die wirksam das ästhetische Erscheinungsbild der Haut oder der Lippen verbessert.

9. Verfahren nach Anspruch 8, wobei die Zusammensetzung auf die Haut aufgetragen wird und wobei die Verbesserung des ästhetischen Erscheinungsbilds aus der Gruppe gewählt wird, die aus Folgendem besteht: die Gesichtsfalten weniger wahrnehmbar machen, bewirken, dass sich die Gesichtsfalten und/oder Runzeln praller anfühlen, das Erscheinungsbild der Falten unter den Augen und/oder der Falten um die Augen verbessern, das Erscheinungsbild der Krähenfüße verbessern, das Auftreten von Falten reduzieren und/oder schwächen, die Haut verjüngen und/oder revitalisieren, die Festigkeit und/oder Prallheit der Haut verbessern, die Spannkraft der Haut verbessern, die Dicke der Haut verstärken, die Größe der Poren verringern, die Feuchtigkeit und/oder Hydratation der Haut verbessern, die Textur der Haut verbessern, feine und/oder tiefe Falten reduzieren und/oder eliminieren, die Haut glätten, den Glanz und/oder die Leuchtkraft der Haut wiederherstellen und die Spannkraft, Geschmeidigkeit und/oder Elastizität der Haut verbessern.

10. Verfahren nach Anspruch 8, wobei die Zusammensetzung auf die Haut aufgetragen wird und wobei die Verbesserung des ästhetischen Erscheinungsbilds die Behandlung mindestens eines Zustands ist, der aus der Gruppe bestehend aus dermatologischer Alterung, Brüchigkeit der Haut, Verlust von Kollagen und/oder Elastin, Atrophie der Haut, Trockenheit der Haut, Abschuppen der Haut, Herabhängen der Haut, Verfärbung der Haut, Anzeichen von Hautermüdung und/oder Stress gewählt wird.

11. Verfahren nach Anspruch 10, wobei die dermatologische Alterung aus der Gruppe bestehend aus chronologischer Alterung, aktinischer Alterung, hormonaler Alterung oder deren Kombinationen gewählt wird.

12. Verfahren nach Anspruch 8, wobei die Haut eine empfindliche Haut ist.

13. Verfahren nach Anspruch 8, wobei das Erscheinungsbild der Haut im Wesentlichen frei von Hautreizungen ist.

14. Verfahren nach Anspruch 8, wobei die Zusammensetzung auf die Lippen aufgetragen wird und wobei die Verbesserung des ästhetischen Erscheinungsbilds der Lippen mindestens ein Element der Gruppe einschließt, die aus Folgendem besteht: die Anzeichen der chronologischen/lichtbedingten Alterung reduzieren, verbessern und/oder behandeln, indem das Erscheinungsbild runzliger Lippen verbessert wird; die Brüchigkeit des Epithels der Lippen verbessern; den Verlust an Kollagen verringern, verhindern und/oder rückgängig machen; der Atrophie des Epithels der Lippen vorbeugen; den Zellumsatz fördern/beschleunigen; die Festigkeit/Prallheit der Lippen/des Epithels verbessern; die Textur der Lippen/des Epithels verbessern; das Auftreten feiner Lippenfalten, insbesondere feiner vertikaler Lippenfalten, verringern/minimieren; die Größe und/oder Tiefe der Runzeln, insbesondere vertikaler Runzeln der Lippen, verringern; die Spannkraft der Lippen/des Epithels verbessern; die Dicke der Lippen/des Epithels erhöhen; die Feuchtigkeitsretention verbessern; die Verfärbung der Lippen/des Epithels minimieren und die altersbedingte Verhornung des Lippenepithels rückgängig machen.

15. Verfahren nach Anspruch 8, wobei die Zusammensetzung auf die Lippen aufgetragen wird und wobei die Verbesserung des ästhetischen Erscheinungsbilds der Lippen mindestens ein Element der Gruppe einschließt, die aus Folgendem besteht: die Anzeichen der chronologischen/lichtbedingten Alterung reduzieren, verbessern und/oder behandeln, indem das Erscheinungsbild runzliger Lippen verbessert wird; die Festigkeit/Prallheit der Lippen/des Epithels verbessern; die Textur der Lippen/des Epithels verbessern; das Auftreten feiner vertikaler Lippenfalten verringern/minimieren; die Größe und/oder Tiefe vertikaler Runzeln der Lippen verringern; die Spannkraft der Lippen/des Epithels verbessern; die Dicke der Lippen/des Epithels erhöhen; die Feuchtigkeitsretention verbessern und die Verfärbung der Lippen/des Epithels minimieren.

16. Verfahren zur Verbesserung des ästhetischen Erscheinungsbilds der Haut, umfassend das topische Auftragen einer im Wesentlichen wasserfreien Zusammensetzung mit Retinoxytrimethylsilan auf die Haut in einer Menge und über einen Zeitraum, die bzw. der ausreichend ist, um das ästhetische Erscheinungsbild der Haut zu verbessern.

17. Verfahren nach Anspruch 16, wobei das Auftragen der Zusammensetzung einmal täglich oder zweimal täglich für bis zu zwei oder vier Wochen erfolgt.

18. Topische, im Wesentlichen wasserfreie Zusammensetzung, umfassend: Retinoxytrimethylsilan in einer Menge, die wirksam die feinen Falten und/oder Runzeln verhindert, verbessert, reduziert und/oder behandelt.

19. Zusammensetzung nach Anspruch 18, wobei Retinoxytrimethylsilan in einer Menge von etwa 0,1 Gew.% bis etwa 50 Gew.% des Gesamtgewichts der topischen Zusammensetzung vorhanden ist.

20. Zusammensetzung nach Anspruch 18, wobei Retinoxytrimethylsilan in einer Menge von etwa 0,5 Gew.% bis etwa 15 Gew.% des Gesamtgewichts der Zusammensetzung vorhanden ist.

21. Zusammensetzung nach Anspruch 18, wobei Retinoxytrimethylsilan in einer Menge von etwa 0,5 Gew.% bis etwa 5 Gew.% des Gesamtgewichts der Zusammensetzung vorhanden ist.

22. Zusammensetzung nach Anspruch 18, die darüber hinaus einen kosmetisch akzeptablen Träger enthält.

23. Zusammensetzung nach Anspruch 18, die darüber hinaus ein Oxidationsschutzmittel enthält.

24. Zusammensetzung nach Anspruch 18, die darüber hinaus ein Sonnenschutzmittel enthält.

25. Zusammensetzung nach Anspruch 18, wobei die Zusammensetzung eine Zusammensetzung für die Lippen ist und Retinoxytrimethylsilan in einer Menge vorhanden ist, die wirksam feine Falten und/oder Runzeln verhindert, verbessert, reduziert und/oder behandelt.

26. Zusammensetzung nach Anspruch 25, wobei die Zusammensetzung für die Lippen mindestens etwa 0,01 Gew.% Retinoxytrimethylsilan enthält.

27. Zusammensetzung nach Anspruch 25, wobei die Zusammensetzung für die Lippen mindestens etwa 0,015 Gew.% Retinoxytrimethylsilan enthält.

28. Zusammensetzung nach Anspruch 25, wobei die Zusammensetzung für die Lippen mindestens etwa 0,02 Gew.% Retinoxytrimethylsilan enthält.

29. Zusammensetzung nach Anspruch 25, wobei die Zusammensetzung für die Lippen weniger als etwa 0,75 Gew.% Retinoxytrimethylsilan enthält.

30. Zusammensetzung nach Anspruch 25, wobei die Zusammensetzung für die Lippen weniger als etwa 0,50 Gew.% Retinoxytrimethylsilan enthält.

31. Verwendung von Retinoxytrimethylsilan zur Herstellung einer im Wesentlichen wasserfreien Zusammensetzung mit Retinoxytrimethylsilan, wobei die Zusammensetzung auf die Haut aufgetragen wird und wobei die Verbesserung des ästhetischen Erscheinungsbilds der Haut die Behandlung mindestens eines Zustands ist, der aus Akne, Psoriasis, Ekzem und Warzen gewählt wird.

## Revendications

1. Procédé de traitement de plis fins et/ou de rides, comprenant l'application à la peau d'une composition topique sensiblement anhydride ayant une quantité de rétinoxytriméthylsilane efficace pour prévenir, améliorer et/ou réduire les plis fins et/ou la formation de rides.

2. Procédé selon la revendication 1, dans lequel ladite composition est appliquée pendant une période efficace pour prévenir, améliorer et/ou réduire les plis fins et/ou la formation de rides.

3. Procédé selon la revendication 2, dans lequel ladite application de la composition s'effectue une ou deux fois par jour sur une période allant jusqu'à deux ou quatre semaines.

4. Procédé selon la revendication 1, dans lequel ledit rétinoxytriméthylsilane est présent en une quantité d'environ 0,1% en poids à environ 50% en poids du poids total de la composition.

5. Procédé selon la revendication 1, dans lequel ledit rétinoxytriméthylsilane est présent en une quantité d'environ 0,5% en poids à environ 15% en poids du poids total de la composition.

6. Procédé selon la revendication 1, dans lequel ledit rétinoxytriméthylsilane est présent en une quantité d'environ 0,5% en poids à environ 5% en poids du poids total de la composition.

7. Procédé selon la revendication 1, dans lequel ladite composition comprend en outre un véhicule cosmétiquement acceptable.

8. Procédé d'amélioration de l'apparence esthétique de la peau ou de la lèvre comprenant l'application par voie topique à la peau ou à la lèvre d'une composition sensiblement anhydride comportant du rétinoxytriméthylsilane en une quantité efficace pour améliorer l'apparence esthétique de la peau ou de la lèvre.

9. Procédé selon la revendication 8, dans lequel la composition est appliquée à la peau et dans lequel l'amélioration en apparence esthétique est choisie dans le groupe consistant à rendre les plis faciaux moins perceptibles, à rendre les plis faciaux et/ou les rides arrondis, à améliorer l'apparence des plis sous-orbitaires et/ou des plis périorbitaires, à améliorer l'apparence des pâtes d'oie, à réduire et/ou diminuer l'apparence des rides, à rajeunir et/ou revitaliser la peau, à améliorer la fermeté et/ou la rondeur de la peau, à améliorer le teint, à accroître l'épaisseur de la peau, à diminuer la grosseur de pore, à améliorer l'humidité et/ou l'hydratation de la peau, à améliorer la texture de la peau, à réduire et/ou éliminer les plis fins et/ou profonds, à lisser la peau, à rétablir l'éclat et/ou la luminosité de la peau et à améliorer la résilience, la flexibilité et/ou l'élasticité de la peau.

10. Procédé selon la revendication 8, dans lequel la composition est appliquée à la peau et dans lequel l'amélioration en apparence esthétique de la peau est le traitement d'au moins un état choisi dans le groupe consistant en le vieillissement dermatologique, la fragilité de la peau, la perte de collagène et/ou d'élastine, l'atrophie de la peau, la sécheresse de la peau, le caractère écailleux de la peau, l'affaissement de la peau, le changement de couleur de la peau, les signes de fatigue et/ou de stress de la peau.

11. Procédé selon la revendication 10, dans lequel ledit vieillissement dermatologique est choisi dans le groupe consistant en le vieillissement chronologique, le vieillissement actinique, le vieillissement hormonal ou toute combinaison de ceux-ci.

12. Procédé selon la revendication 8, dans lequel ladite peau est une peau sensible.

13. Procédé selon la revendication 8, dans lequel ladite apparence de la peau est sensiblement dépourvue d'irritation de la peau.

14. Procédé selon la revendication 8, dans lequel la composition est appliquée à la lèvre et dans lequel l'amélioration en apparence esthétique de la lèvre comprend au moins l'un choisi dans le groupe consistant en réduire, améliorer et/ou traiter les signes du vieillissement chronologique/photovieillissement en améliorant l'apparence des lèvres ridées ; diminuer la fragilité épithéliale de la lèvre ; améliorer, anticiper et/ou inverser la perte de collagène ; prévenir l'atrophie épithéliale de la lèvre ; promouvoir/accélérer le renouvellement cellulaire ; améliorer la fermeté/rondeur de la lèvre/épithéliale ; améliorer la texture de la lèvre/épithéliale ; diminuer/minimiser l'apparence des plis fins de la lèvre, en particulier les plis fins verticaux de la lèvre ; diminuer la taille et/ou la profondeur des rides, en particulier les rides verticales de la lèvre ; améliorer la teinte de la lèvre/épithéfiale ; accroître l'épaisseur de la lèvre/épithéliale ; augmenter la rétention d'humidité ; minimiser le changement de couleur de la lèvre/épithéliale ; et inverser la kératinisation associée à l'âge de l'épithélium de la lèvre.

15. Procédé selon la revendication 8, dans lequel la composition est appliquée à la lèvre et dans lequel l'amélioration en apparence esthétique de la lèvre comprend au moins un élément du groupe consistant en réduire, améliorer et/ou traiter les signes du vieillissement chronologique/photovieillissement en améliorant l'apparence des lèvres ridées ; améliorer la fermeté/rondeur de la lèvre/épithéliale ; améliorer la texture de la lèvre/épithéliale ; diminuer/minimiser l'apparence des plis fins verticaux de la lèvre ; diminuer la taille et/ou la profondeur des rides verticales de la lèvre ; améliorer la teinte de la lèvre/épithéliale ; accroître l'épaisseur de la lèvre/épithéliale ; augmenter la rétention d'humidité ; et minimiser le changement de couleur de la lèvre/épithéliale.

16. Procédé d'amélioration de l'apparence esthétique de la peau comprenant : l'application par voie topique à la peau d'une composition sensiblement anhydride comportant du rétinoxytriméthylsilane, en une quantité et pendant une période suffisantes pour améliorer l'apparence esthétique de la peau.

17. Procédé selon la revendication 16, dans lequel ladite application de la composition s'effectue une ou deux fois par jour sur une période allant jusqu'à deux ou quatre semaines.

18. Composition topique sensiblement anhydride, comprenant : du rétinoxytriméthylsilane en une quantité efficace pour prévenir, améliorer, réduire et/ou traiter des plis fins et/ou des rides.

19. Composition selon la revendication 18, dans laquelle ledit rétinoxytriméthylsilane est présent en une quantité d'environ 0,1% à environ 50% en poids du poids total de la composition.

20. Composition selon la revendication 18, dans laquelle ledit rétinoxytriméthylsilane est présent en une quantité d'environ 0,5% à environ 15% en poids du poids total de la composition topique.

21. Composition selon la revendication 18, dans laquelle ledit rétinoxytriméthylsilane est présent en une quantité d'environ 0,5% en poids à environ 5% en poids du poids total de la composition.

22. Composition selon la revendication 18; comprenant en outre un véhicule cosmétiquement acceptable.

23. Composition selon la revendication 18, comprenant en outre un antioxydant.

24. Composition selon la revendication 18, comprenant en outre un écran solaire.

25. Composition selon la revendication 18, dans laquelle la composition est une composition pour lèvres et ledit rétinoxytriméthylsilane est présent en une quantité efficace pour prévenir, améliorer, réduire et/ou traiter des plis fins et/ou des rides.

26. Composition selon la revendication 25, dans laquelle ladite composition pour lèvres comprend au moins environ 0,01% en poids dudit rétinoxytriméthylsilane.

27. Composition selon la revendication 25, dans laquelle ladite composition pour lèvres comprend au moins environ 0,015% en poids dudit rétinoxytriméthylsilane.

28. Composition selon la revendication 25, dans laquelle ladite composition pour lèvres comprend au moins environ 0,02% en poids dudit rétinoxytriméthylsilane.

29. Composition selon la revendication 25, dans laquelle ladite composition pour lèvres comprend au moins environ 0,75% en poids dudit rétinoxytriméthylsilane.

30. Composition selon la revendication 25, dans laquelle ladite composition pour lèvres comprend au moins environ 0,50% en poids dudit rétinoxytriméthylsilane.

31. Utilisation de rétinoxytriméthylsilane pour la fabrication d'une composition sensiblement anhydride comportant du rétinoxytriméthylsilane où la composition est appliquée à la peau et où l'amélioration en apparence esthétique de la peau est le traitement d'au moins un état choisi parmi l'acné, le psoriasis, l'eczéma et les verrues.
